# EUROPEAN PATENT APPLICATION

(11) **EP 2 065 473 A1**
(43) Date of publication of application: **03.06.2009**
(21) Application number: 07121803.6
(22) Date of filing: 28.11.2007
(51) Int. Cl.: C12Q 1/68

(54) **A method to assess prognosis and to predict therapeutic success in gynecologic cancer**

(71) Applicant: Siemens Healthcare Diagnostics GmbH, 65760 Eschborn (DE)
(72) Inventor: Darb-Esfahani, Silvia, Dr., 12169 Berlin (DE); Denkert, Carsten, Dr., 10405 Berlin (DE); Dietel, Manfred, Professor, 14193 Berlin (DE); Sinn, Bruno, 10115 Berlin (DE); Martoni, Andrea, Professor, 40136 Bologna (IT); Zamagni, Caludio, Dr., 40132 Bologna (IT); Wirtz, Ralph, Markus, Dr., 50677 Köln (DE)
(74) Representative: Maier, Daniel Oliver

(57) **Abstract**

The present invention is related to a method for predicting a clinical response of a patient suffering from or at risk of developing a gynecologic cancer towards a given mode of treatment, said method comprising the steps of:
a) obtaining a biological sample from said patient;
b) determining, on a non protein basis, the expression level of at least one gene encoding for a hormone receptor selected from the group comprising estrogen receptor, progesterone receptor and/or androgen receptor in said sample;
c) comparing the pattern of expression level(s) determined in step b) with one or several reference pattern(s) of expression levels; and
d) predicting therapeutic success for said given mode of treatment in said patient from the outcome of the comparison in step c).

## Description

### Field of the invention

The present invention relates to a method for prognosticating and predicting therapeutic outcome in cancer treatment.

### Background of the invention

Ovarian cancer is the most lethal gynecologic cancer with 20,000 new cases per year and 15,000 deaths per year in the US. Despite prominent responses towards chemotherapy, the survival of ovarian cancer patients is still very poor.

Chemotherapy is standard of care for early and advanced ovarian cancer. However, there are only few data addressing the molecular prediction of response to therapy in ovarian cancer.

It is a well established fact, that systemic treatment after surgery reduces the risk of disease relapse and death in patients with primary operable ovarian cancer. However, there still is a great number of patients who do not benefit from systemic therapy, which is reflected by the fact, that about 70% of patients being diagnosed to suffer ovarian cancer will die due to this disease. Consequently ovarian carcinoma is the fifth leading cause of cancer-related deaths in women. The 5-year survival rate for patients diagnosed with stage I disease is nearly 94%; however, because patients with ovarian cancer typically present with nonspecific symptoms, the disease is frequently diagnosed at late stages (III or IV) when it has spread to the upper abdomen or beyond and the 5-year survival rate drops below 30%.

In general, ovarian cancer patients do receive the similar standard regimen, i.e. platinum-based chemotherapy after extensive surgery aiming to achieve R0 resection of the tumor. However, despite high initial response rates of ovarian tumors towards chemotherapeutic regimen and benefit from optimal surgical cytoreduction, a large proportion of patients with advanced ovarian cancer suffers relapse and remain to have a poor clinical outcome. Because these patients are not curable, the goal of therapy becomes improvement of both quality and length of life. However, chemotherapeutic regimen result in pronounced side effects. Also the inclusion of new drugs, such as the vascular endothelial growth factor A (VEGFA) antibody bevacicumab (trademname Avastin®) results in serious side effects, e.g. 1 to 6% develop gastrointestinal perforation, which may be lethal.

Markers predicting tumor response can function as sensitive short-term surrogates of longterm outcome. Response to primary chemotherapy is an excellent experimental model to study the efficacy of anticancer therapy in a relatively short period of time. Moreover, the molecular analysis of pre- and post-chemotherapy tumor specimen enables the identification of chemotherapy resistant tumor cell subpopulation and thereby leads to adapted treatment options. However the identification of relevant resistance mechanisms in such settings and development of tests that could be used to detect these underlying resistance mechanisms for patient selection before therapy in clinical routine tissue have not succeeded so far. The use of such markers can make therapeutic strategies more effective for the individual patient and will allow changing regimen early in the case of non-responding tumors. Moreover, the identification of such markers has the potential to identify new drug targets and develop new and more effective treatments.

Interestingly, while hormonal therapies are commonly used in the treatment of endocrine organ-associated malignancies such as breast and prostate cancer, at present time neither hormonal therapy is approved by the European Agency for the Evaluation of Medicinal Products (EMEA) or by the US Food and Drug Administration (FDA) for the treatment of epithelial ovarian carcinoma nor has a diagnostic marker been identified, that could select patients for endocrine options.

Although much effort has been devoted in developing an optimal clinical treatment course for individual patients with ovarian cancer, only little progress has been made in predicting the individual's response to a certain treatment. Particularly, no stratification marker has been validated so far that is useful to adopt the primary systemic treatment, e.g. by including endocrine options in the adjuvant setting.

Currently, the probability of response of patients to a certain cancer treatment is usually determined by measuring the status of a marker on protein level by immunohistochemistry (IHC). Assays based on protein-level measurements exhibit only limited quantitative performance and comparatively high inter- and intra- assay variabilities. Especially immunohistochemistry often yields different results in different laboratories.

Other approaches, as FISH (Fluorescence In Situ Hybridization) or expression profiling analysis, suffer of drawbacks as low sensitivity, restriction of sample preparation and restricted multiplexing capabilities.

To date there are no reliable response markers to predict response to cancer therapy, neither to platinum-based chemotherapy nor to endocrine treatment options, in ovarian cancer based on immunohistochemistry, FISH or expression profiling analysis.

So, it is yet difficult to determine those patients suffering of ovarian cancer who will respond to a certain therapy.

Therefore, a need exists to identify those patients who are likely to respond to therapy by providing predictive information.

### Definitions

Unless defined otherwise, technical and scientific terms used herein have the same meaning as commonly understood by one of ordinary skill in the art to which this invention belongs.

The term "prediction" as used herein relates to the likelihood that a patient will respond either favorably or unfavorably to a given therapy. Especially, the term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assesment of the malignancy of a tumor, or to the expected survival rate (DFS, disease free survival) of a patient, if the tumor remains untreated.

The term "response marker" relates to a marker which can be used to predict the clinical response of a patient towards a given treatment. Response includes direct observation of tumor shrinkage upon neoadjuvant or palliative treatment as evident by e.g. CT-Scans and/or serum biomarkers as well as effects on Disease Free Survival (DFS), Overall Survival (OAS), Metastasis Specific Survival (MSS), Disease Specific Survival and related assessments.

The term "clinical response" of a patient, as used herein, relates to the effectiveness of a certain therapy in a patient, meaning an improvement in any measure of patient status, including those measures ordinarily used in the art, such as overall survival, progression free survival, recurrence-free survival, and distant recurrence-free survival. Recurrence-free survival (RFS) refers to the time (in years) from surgery to the first local, regional, or distant recurrence. Distant recurrence-free survival (DFRS) refers to the time (in years) from surgery and/or initial diagnosis to the first anatomically distant recurrence. The calculation of these measures in practice may vary from study to study depending on the definition of events to be either censored or not considered. The term "response marker" relates to a marker which can be used to predict the clinical response of a patient towards a given treatment.

The term "neoplastic disease" refers to a cancerous tissue this includes carcinomas, e.g., carcinoma in situ, invasive carcinoma, metastatic carcinoma, and pre-malignant conditions, neomorphic changes independent of their histological origin, e.g. papillary serous, mucinous, endometriod, clear cell, ductal, lobular, medullary, mixed origin.

The terms "cancer" and "cancerous" refer to or describe the physiological condition in mammals that is typically characterized by unregulated cell growth. The term "cancer" as used herein includes carcinomas, (e.g., carcinoma in situ, invasive carcinoma, metastatic carcinoma) and pre-malignant conditions, blood cell neoplasms and neomorphic changes independent of their histological origin. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer, primary carcinomas, and all other types of cancers, malignancies and transformations specially associated with gynecologic cancer are included. The terms "neoplastic disease" or "cancer" are not limited to any tissue or cell type they also include primary, secondary or metastatic lesions of cancer patients, and also comprise lymph nodes affected by cancer cells or minimal residual disease cells either locally deposited or freely floating throughout the patients body.

As used herein, the term "gynecologic cancers" refers to cancer which are diagnosed in female breast and reproductive organs that include the uterus, ovaries, cervix, fallopian tubes, vulva, and vagina. Examples of gynecologic cancers include, but are not limited to breast cancer, ovarian cancer, vulvar cancer, vaginal cancer, tubal cancer, endometrian cancer and/or cervical cancer. As used herein, the term "endometrian cancer", also called endometrial cancer or uterine cancer, includes malignant growth of cells in the endometrium, the lining of the uterus.

The term "tumor" as used herein, refers to all neoplastic cell growth and proliferation, whether malignant or benign, and all pre-cancerous and cancerous cells and tissues.

The term "neoplastic cells" refer to abnormal cells that grow by increased cellular proliferation, altered cell division symmetry or decreased cell death mechanisms more rapidly than normal. As such, neoplastic cells of the invention may be cells of a benign neoplasm or may be cells of a malignant neoplasm.

Furthermore, the term "characterizing the state of a neoplastic disease" is related to, but not limited to, measurements and assessment of one or more of the following conditions: Type of tumor, histomorphological appearance, dependence on external signal (e.g. hormones, growth factors), invasiveness, motility, state by TNM Classification of Malignant Tumours (TNM), a cancer staging system developed and maintained by the International Union Against Cancer, or similar, agressivity, malignancy, metastatic potential, and responsiveness to a given therapy.

The term "therapy modality", "therapy mode", "regimen" or "chemo regimen" as well as "therapy regimen" refers to a timely sequential or simultaneous administration of antitumor, and/or anti vascular, and/or immune stimulating, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of the single agent, time-frame of application and frequency of administration within a defined therapy window. Currently various combinations of various drugs and/or physical methods, and various schedules are under investigation.

The term "endocrine treatment" refers to various treatment modalities known as hormonal therapy or anti hormonal therapy that produce the desired therapeutic effect by means of change of hormone/hormones level. The treatment may include administration of hormones or hormone analogs, synthetic hormones or other drugs to the patient, or decreasing the level of hormones in the body by using hormone antagonists, or hormone ablation therapy either by surgical resection of ovaries or by chemical suppression of hormone synthesis. Endocrine therapy shall be taken to include hormonal therapies such as selective estrogen reuptake inhibitors, selective estrogen receptor downregulators, aromatase inhibitors and ovarian ablation.

The term "determining the expression level of a gene on a non protein basis" relates to methods which are not focussed on the secondary gene translation products, i.e proteins, but on other levels of the gene expression, based on RNA and DNA analysis. In one embodiment of this invention the analysis uses mRNA including its precursor forms. An exemplary determinable property is the amount of the estrogen receptor or progesterone receptor RNA, i.e. of the ESR1, ESR2 and/or PGR gene.

The term "expression level" refers, e.g., to a determined level of gene expression. The term "pattern of expression levels" refers to a determined level of gene expression compared either to a reference gene, e.g. housekeeper, or inversely regulated genes, or to a computed average expression value, e.g. in DNA-chip analyses. A pattern is not limited to the comparison of two genes but is more related to multiple comparisons of genes to reference genes or samples. A certain "pattern of expression levels" may also result and be determined by comparison and measurement of several genes disclosed hereafter and display the relative abundance of these transcripts to each other.

Alternatively, a differentially expressed gene disclosed herein may be used in methods for identifying reagents and compounds and uses of these reagents and compounds for the treatment of cancer as well as methods of treatment. The differential regulation of the gene is not limited to a specific cancer cell type or clone, but rather displays the interplay of cancer cells, muscle cells, stromal cells, connective tissue cells, other epithelial cells, fat cells, endothelial cells of blood vessels as well as cells of the immune system, e.g. lymphocytes, macrophages, killer cells.

The term "RNA expression level" refers to a determined level of the converted DNA gene sequence information into transcribed RNA, the initial unspliced RNA transcript or the mature mRNA. RNA expression can be monitored by measuring the levels of either the entire RNA of the gene or subsequences.

The term "pattern of RNA expression" refers to a determined level of RNA expression compared either to a reference RNA or to a computed average expression value. A pattern is not limited to the comparison of two RNAs but is more related to multiple comparisons of RNAs to reference RNAs or samples. A certain "pattern of expression levels" may also result and be determined by comparison and measurement of several RNAs and display the relative abundance of these transcripts to each other.

A "reference pattern of expression levels", within the meaning of the invention shall be understood as being any pattern of expression levels that can be used for the comparison to another pattern of expression levels. In a preferred embodiment of the invention, a reference pattern of expression levels is, e.g., an average pattern of expression levels observed in a group of healthy or diseased individuals, serving as a reference group.

The terms "biological sample" or "clinical sample", as used herein, refer to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, liquor cerebrospinalis, tear fluid, or cells there from. Biological samples may also include sections of tissues such as frozen or fixed sections taken for histological purposes or microdissected cells or extracellular parts thereof. A biological sample to be analyzed is tissue material from a neoplastic lesion taken by aspiration or punctuation, excision or by any other surgical method leading to biopsy or resected cellular material. Such a biological sample may comprise cells obtained from a patient. The cells may be found in a cell "smear" collected, for example, by a nipple aspiration, ductal lavarge, fine needle biopsy or from provoked or spontaneous nipple discharge. In another embodiment, the sample is a body fluid. Such fluids include, for example, blood fluids, serum, plasma, lymph, ascitic fluids, gynecologic fluids, or urine but not limited to these fluids.

By "array" is meant an arrangement of addressable locations or "addresses" on a device. The locations can be arranged in two dimensional arrays, three dimensional arrays, or other matrix formats. The number of locations can range from several to at least hundreds of thousands. Most importantly, each location represents an independent reaction site. Arrays include but are not limited to nucleic acid arrays, protein arrays and antibody arrays. A "nucleic acid array" refers to an array containing nucleic acid probes, such as oligonucleotides, polynucleotides or larger portions of genes. The nucleic acid on the array is preferably single stranded. Arrays wherein the probes are oligonucleotides are referred to as "oligonucleotide arrays" or "oligonucleotide chips." A "microarray," herein also refers to a "biochip" or "biological chip", an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides are preferably single-stranded DNA probe oligonucleotides. Moreover, in context of applicable detection methodologies, the term "oligonucleotide" also refers to nucleotide analogues such as PNAs and morpholinos.

The terms "modulated" or "modulation" or "regulated" or "regulation" and "differentially regulated" as used herein refer to both upregulation, i.e., activation or stimulation, e.g., by agonizing or potentiating, and down regulation, i.e., inhibition or suppression, e.g., by antagonizing, decreasing or inhibiting.

"Primer pairs" and "probes", within the meaning of the invention, shall have the ordinary meaning of this term which is well known to the person skilled in the art of molecular biology. In a preferred embodiment of the invention "primer pairs" and "probes", shall be understood as being polynucleotide molecules having a sequence identical, complementary, homologous, or homologous to the complement of regions of a target polynucleotide which is to be detected or quantified. In yet another embodiment, nucleotide analogues are also comprised for usage as primers and/or probes. Probe technologies used for kinetic or real time PCR applications could be e.g. TaqMan® systems obtainable at Roche Molecular Diagnostics, extension probes such as Scorpion® Primers, Dual Hybridisation Probes, Amplifluor® obtainable at Chemicon International, Inc, or Minor Groove Binders.

"Individually labeled probes", within the meaning of the invention, shall be understood as being molecular probes comprising a polynucleotide, oligonucleotide or nucleotide analogue and a label, helpful in the detection or quantification of the probe. Preferred labels are fluorescent molecules, luminescent molecules, radioactive molecules, enzymatic molecules and/or quenching molecules.

"Arrayed probes", within the meaning of the invention, shall be understood as being a collection of immobilized probes, preferably in an orderly arrangement. In a preferred embodiment of the invention, the individual "arrayed probes" can be identified by their respective position on the solid support, e.g., on a "chip".

The phrase "response", "therapeutic success", or "response to therapy" refers in the neoadjuvant, adjuvant and palliative chemotherapeutic setting to the observation of a defined tumor free or recurrence free or progression free survival time (e.g. 2 years, 4 years, 5 years, 10 years). This time period of disease free -, recurrence free - or progression free survival may vary among the different tumor entities but is sufficiently longer than the average time period in which most of the recurrences appear. In a neoadjuvant and palliative therapy modality, response may additionally be monitored by measurement of tumor shrinkage and regression due to apoptosis and necrosis of the tumor mass or reduced blood supply due to altered angiogenic events.

The term "recurrence" or " recurrent disease" includes distant metastasis that can appear even many years after the initial diagnosis and therapy of a tumor, or local events such as infiltration of tumor cells into regional lymph nodes, or occurrence of tumor cells at the same site and organ of origin within an appropriate time.

"Prediction of recurrence" or "prediction of therapeutic success" does refer to the methods described in this invention, wherein a tumor specimen is analyzed for e.g. its gene expression, genomic status and/or histopathological parameters (such as TNM and Grade) and/or imaging data and furthermore classified based on correlation of the expression pattern to known ones from reference samples. This classification may either result in the statement that such given tumor will develop recurrence and therefore is considered as a "non responding" tumor to the given therapy, or may result in a classification as a tumor with a prolonged disease free post therapy time.

"Biological activity" or "bioactivity" or "activity" or "biological function", which are used interchangeably, herein mean an effector or antigenic function that is directly or indirectly exerted by a polypeptide (whether in its native or denatured conformation), or by any fragment thereof *in vivo* or *in vitro.* Biological activities include but are not limited to binding to polypeptides, binding to other proteins or molecules, enzymatic activity, signal transduction, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "marker" or "biomarker" refers to a biological molecule, e.g., a nucleic acid, peptide, protein, hormone, etc., whose presence or concentration can be detected and correlated with a known condition, such as a disease state or a combination of these, e.g. by a mathematical algorithm.

The term "marker gene" as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and gynecological cancer in particular. A marker gene may also have the characteristics of a target gene.

"Target gene", as used herein, refers to a differentially expressed gene involved in cancer, e.g. gynecologic cancer, preferably ovarian cancer, in a manner in which modulation of the level of the target gene expression or of the target gene product activity may act to ameliorate symptoms of malignant neoplasia. A target gene may also have the characteristics of a marker gene.

The term "receptor", as used herein, relates to a protein on the cell membrane or within the cytoplasm or cell nucleus that binds to a specific molecule (a ligand), such as a neurotransmitter, hormone, or other substance, especially a hormone as estrogen, and initiates the cellular response. Ligand-induced changes in the behavior of receptor proteins result in physiological changes that constitute the biological actions of the ligands.

The term "signalling pathway" is related to any intra- or intercellular process by which cells converts one kind of signal or stimulus into another, most often involving ordered sequences of biochemical reactions out- and inside the cell, that are carried out by enzymes and linked through hormones and growth factors (intercellular), as well as second messengers (intracellular), the latter resulting in what is thought of as a "second messenger pathway". In many signalling pathways, the number of proteins and other molecules participating in these events increases as the process emanates from the initial stimulus, resulting in a "signal cascade" and often results in a relatively small stimulus eliciting a large response.

The term "small molecule", as used herein, is meant to refer to a compound which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

When used in reference to a single-stranded nucleic acid sequence, the term "substantially homologous" refers to any probe that can hybridize (i.e., it is the complement of) the single-stranded nucleic acid sequence under conditions of low stringency as described above.

As used herein, the term "hybridization" is used in reference to the pairing of complementary nucleic acids.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described below. When it comes to the determination of expression levels, hybridization based methods may for example be used to determine the amount of mRNA for a given gene.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). The term "PCR based method" comprises both end-point PCR applications as well as kinetic/real time PCR techniques applying special fluorophors or intercalating dyes which emit fluorescent signals as a function of amplified target and allow monitoring and quantification of the target. Quantification methods could be either absolute by external standard curves or relative to a comparative internal standard.

The term "method based on the electrochemical detection of molecules" relates to methods which make use of an electrode system to which molecules, particularly biomolecules like proteins, nucleic acids, antigens, antibodies and the like, bind under creation of a detectable signal. Such methods are for example disclosed in WO0242759, WO0241992 and WO02097413 filed by the applicant of the present invention, the content of which is incorporated by reference herein. These detectors comprise a substrate with a planar surface which is formed, for example, by the crystallographic surface of a silicon chip, and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules. The probe molecules are for example immobilized by a Thiol-Gold-binding. For this purpose, the probe is modified at its 5'-or 3'-end with a thiol group which binds to the electrode comprising a gold surface. These target nucleic acid molecules may carry, for example, an enzyme label, like horseradish peroxidase (HRP) or alkaline phosphatase. After the target molecules have bound to the probes, a substrate is then added (e.g., α-naphthyl phosphate or 3,3'5,5'-tetramethylbenzidine which is converted by said enzyme, particularly in a redox-reaction. The product of said reaction, or a current generated in said reaction due to an exchange of electrons, can then be detected with help of the electrical detector in a site specific manner.

The term "nucleic acid molecule" is intended to indicate any single- or double stranded nucleic acid and/or analogous molecules comprising DNA, cDNA and/or genomic DNA, RNA, preferably mRNA, peptide nucleic acid (PNA), locked nucleic acid (LNA) and/or Morpholino.

The term "stringent conditions" relates to conditions under which a probe will hybridize to its target subsequence, but to no other sequences. Stringent conditions are sequence-dependent and will be different in different circumstances. Longer sequences hybridize specifically at higher temperatures. Generally, stringent conditions are selected to be about 5° C. lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. (As the target sequences are generally present in excess, at Tm, 50% of the probes are occupied at equilibrium). Typically, stringent conditions will be those in which the salt concentration is less than about 1.0 M Na ion, typically about 0.01 to 1.0 M Na ion (or other salts) at pH 7.0 to 8.3 and the temperature is at least about 30° C. for short probes (e.g. 10 to 50 nucleotides) and at least about 60° C. for longer probes. Stringent conditions may also be achieved with the addition of destabilizing agents, such as formamide and the like.

The term "fragment of the nucleic acid molecule" is intended to indicate a nucleic acid comprising a subset of a nucleic acid molecule according to one of the claimed sequences.The same is applicable to the term "fraction of the nucleic acid molecule".

The term "variant of the nucleic acid molecule" refers herein to a nucleic acid molecule which is substantially similar in structure and biological activity to a nucleic acid molecule according to one of the claimed sequences.

The term "homologue of the nucleic acid molecule" refers to a nucleic acid molecule the sequence of which has one or more nucleotides added, deleted, substituted or otherwise chemically modified in comparison to a nucleic acid molecule according to one of the claimed sequences, provided always that the homologue retains substantially the same binding properties as the latter.

The term "derivative" as used herein, refers to a nucleic acid molecule that has similar binding characteristics to a target nucleic acid sequence as a nucleic acid molecule according to one of the claimed sequences

The term "hybridizing counterparts" as used herein, refers to a nucleic acid molecule that is capable of hybridizing to a nucleic acid molecules under stringent conditions.

The term "anamnesis" relates to patient data gained by a physician or other healthcare professional by asking specific questions, either of the patient or of other people who know the person and can give suitable information (in this case, it is sometimes called heteroanamnesis), with the aim of obtaining information useful in formulating a diagnosis and providing medical care to the patient. This kind of information is called the symptoms, in contrast with clinical signs, which are ascertained by direct examination.

The term "etiopathology" relates to the course of a disease, that is its duration, its clinical symptoms, and its outcome.

### Object of the invention

The technical problem underlying the present invention is to provide biological markers allowing to predict outcome of cancer patients by providing prognostic and/or predictive information concerning the therapeutic outcome of a given treatment in gynecologic cancer including surgery, systemic and/or local application of chemotherapeutic and/or endocrine agents as well as antibody based, nucleic acid based and/or small molecule based strategies.

It is another object of the present invention to provide a method for predicting a clinical response of gynaecologic cancer to a given treatment based on tissue analysis before, during or after therapy.

These objects are met with the methods and means according to the independent claims of the present invention. The dependent claims are related to preferred embodiments.

### Summary of the invention

Before the invention is described in detail, it is to be understood that this invention is not limited to the particular component parts of the process steps of the methods described as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

According to the invention, a method is provided for predicting a clinical response of a patient suffering from or at risk of developing a gynecologic cancer, preferably ovarian cancer, towards a given mode of treatment, said method comprising the steps of:
a) obtaining a biological sample from said patient;
b) determining, on a non protein basis, the expression level of at least one gene encoding for a hormone receptor selected from the group comprising estrogen receptor, progesterone receptor and/or androgen receptor in said sample;
c) comparing the pattern of expression level(s) determined in step b) with one or several reference pattern(s) of expression levels; and
d) predicting therapeutic success for said given mode of treatment in said patient from the outcome of the comparison in step c).

In another aspect of the present invention is provided a method for predicting a clinical response of a patient suffering from or at risk of developing gynecologic cancer, preferably ovarian cancer, towards a given mode of treatment, said method comprising the steps of:
a) obtaining a biological sample from said patient;
b) determining, on a non protein basis, the expression level of at least one gene encoding for a hormone receptor selected from the group comprising estrogen receptor, progesterone receptor and/or androgen receptor in said sample;
c) comparing the pattern of expression level(s) determined in step b) with one or several reference pattern(s) of expression levels; and
d) predicting therapeutic success for a therapeutic regimen targeting hormone receptors selected from the group comprising estrogen receptor, progesterone receptor and/or androgen receptor or signalling pathways in said patient from the outcome of the comparison in step c).

By way of illustration and not by way of limitation said signaling activities comprise receptor tyrosine kinase signaling, e.g. via epidermal growth factor receptor (EGFR) family members, vascular endothelial growth factor receptor (VEGFR) signaling, Fibroblast Growth Factor Receptor (FGFR) family members, Platelet Derived Growth Factor Receptor (PDGFR) family members, c-KIT, a proto-oncogene encoding a receptor tyrosine kinase, or Mesenchymal epithelial transition factor (c-Met); WNT signaling; Notch signaling; Hedgehog signaling; Transforming growth factor-beta (TGF-beta) / SMAD signaling and nuclear factor-kappa B (NFkB) signaling.

Up to now the prognostic and predictive role of hormone receptors selected from the group comprising estrogen receptor, progesterone receptor and/or androgen receptor has not been shown. Accordingly no endocrine treatment options are offered to ovarian cancer patients in the neoadjuvant or adjuvant setting. So far, endocrine treatments have been tested only as part of phase II trials after multiple preceding failures of chemotherapeutic strategies.

Surprisingly, the inventors have found that the expression level of a gene encoding for a hormone receptor selected from the group comprising estrogen receptor, progesterone receptor and/or androgen receptor has prognostic and/or predictive value in gynecologic cancer, preferably in ovarian cancer.

In this regard it is to be understood, that the analysis of estrogen receptor and progesterone receptor status on protein basis has turned out to be inferior to the detection of genes coding for estrogen receptor on RNA basis, as the determination of estrogen receptor by immune histochemistry fails to have prognostic value for gynecologic cancer, preferably ovarian cancer. This has been experimentally confirmed by the inventors in a very same cohort of patients, where the diagnostic value of estrogen and/or progesterone receptor expression determination by kinetic PCR (kPCR) methods has been proven.

The analysis of a gene coding for hormone receptors selected from the group comprising estrogen receptor, progesterone receptor and/or androgen receptor especially on RNA level has the advantage to provide an analysis of a gene coding for hormone receptors selected from the group comprising estrogen receptor, progesterone receptor and/or androgen receptor for the first time for predicting a clinical response to a given treatment in patients suffering from gynecologic cancer, preferably ovarian cancer.

The validity of these findings have been shown by independent measurements of fresh tissue biopsies and resectates by array analyis and also by PCR based analysis of clinical routine material, i.e. formalin fixed and paraffin embedded (=FFPE) tissues.

The method may be used to determine whether a patient is likely to respond to a given treatment. This means that, for example, the invention provides the possibility to specifically determine gynecologic cancer, preferably ovarian cancer, in a patient characterized by an altered expression level of a gene coding for a hormone receptor selected from the group comprising estrogen receptor, progesterone receptor and/or androgen receptor. On the basis of this finding it can be selected if the given treatment is the most promising therapy for the respective patient or if treatment modalities should be altered.

Moreover, the inventors suggest, for the first time, to use the expression level of a gene encoding for hormone receptors selected from the group comprising estrogen receptor, progesterone receptor and/or androgen receptor for the decision whether a given therapy is the most promising therapy for the respective patient having gynecologic cancer, preferably ovarian cancer or if treatment modalities should be altered. In particular, the method disclosed herein is highly prognostic in the identical samples of a patient cohort where the state of the art technology, i.e. immunohistochemistry (=IHC), clearly fails to have any prognostic information.

The prediction of therapeutic success or the investigation of the response to a treatment can be performed at time of first biopsy or after surgery, at a stage in which other methods can not provide the required information on the patient's response to chemotherapy. Hence the current invention also provides means to decide even shortly after tumor surgery whether or not a certain mode of chemotherapy is likely to be beneficial to the patient's health and/or whether to maintain or change the applied mode of chemotherapy treatment. This is of particular importance as the decision which systemic therapy to apply first is of outmost importance for survival, development of resistance and therefore also for subsequent treatment strategies. Also the overall status of patients is usually best at initial diagnosis and therefore allows to apply more complex and/or aggressive treatment options at intended. This not only holds true for chemotherapeutic strategies but is also of importance for generally less toxic strategies, such as anti-angiogenic treatments as exemplified by application of Bevacizumab (tradename Avastin®), Sunitinib (tradename Sutent®) or Sorafenib (tradename Nexavar®). The reason for this is in part the extensive surgery being necessary for gynecologic cancer, preferably ovarian cancer, which inter alias increases the risk of bleeding and gastrointestinal perforation.

According to the superiority of non protein based determination of hormone receptor status selected from the group comprising estrogen receptor, progesterone receptor and/or androgen receptor status, the method should substitute currently available measurements, or used in addition to currently available tests or histopathological parameter to make diagnosis more accurate.

Furthermore the method according to the invention may be applied in neoadjuvant, adjuvant and metastatic settings. Importantly, the inventors have found, that hormone receptors such as estrogen, progesterone and /or androgen receptor are useful for prediction based on untreated tumor samples but also prognostic for treated tumor samples. It has been found for the first time, that there are differences between de novo and acquired hormone receptor activities.

The inventors suggest, for the first time, to use the expression level of at least one gene encoding for estrogen receptor for the decision whether a therapeutic regimen targeting the estrogen receptor, e. g. endocrine therapy, could be beneficial in that patient. This particularly not only comprises different kinds of hormone antagonists or enzyme inhibitors blocking steps of the estrogen biosynthesis but also the usage of hormone agonists, e.g. estrogen, as this could accelerate hormonal control of deregulated cancer cell activities and/or sensitize towards other therapeutic options such as chemotherapy.

Moreover, the inventors suggest, for the first time, to use the expression level of at least one gene encoding for progesterone receptor for the decision whether a therapeutic regimen targeting the progesterone receptor, e. g. endocrine therapy, could be beneficial in that patient. This particularly not only comprises different kinds of hormone antagonists or enzyme inhibitors blocking steps of the estrogen biosynthesis but also the usage of hormone agonists, e.g. natural progestin or synthetic gestagens, as this could accelerate hormonal control of deregulated cancer cell activities and/or sensitize towards other therapeutic options such as chemotherapy.

It is yet another embodiment of the present invention to provide a method for predicting the development of resistance to therapeutic intervention of a patient suffering from gynecologic cancer, preferably ovarian cancer to a given treatment.

In this context it is of note that it is part of this invention to use the described method to stratify patients, which may benefit from hormonal treatments, such as estrogen, progestin and gestagen.

It is yet a preferred embodiment of the present invention to provide a method to stratify patients for systemic treatments other than chemotherapy in a neoadjuvant, adjuvant or palliative setting. In a preferred embodiment these alternative treatment options comprise antibody based or small molecule based treatment. However in a most preferred embodiment this relates to endocrine treatment options.

Moreover this finding also enables to decide which patients exhibit good prognosis by receiving chemotherapeutic regimen and which patients should additionally receive endocrine treatments.

Moreover, the method according to the invention may help to detect those tumors which are probably more susceptible to endocrine treatment than to a chemotherapeutic regimen. These tumors have so far remained undetected with methods from the state of the art. Particularly the determination of ESR1 status by IHC is not part of the current standard of care as it does not provide any prognostic information. Thereby the endocrine options have been neglected for treatment of ovarian cancer.

The inventors suggest moreover to use the expression level of at least one gene encoding for estrogen receptor, preferably selected from the group comprising ESR1 and/or ESR2, and/or progesterone receptor, preferably PGR, and/or androgen receptor for the decision whether a therapeutic regimen targeting signalling pathways, preferably as specified above, could be beneficial in that patient.

In a preferred embodiment of the invention, said given mode of chemotherapy is targeted therapy such as small molecule inhibitors like Sunitinib (tradename Sutent®), Sorafenib (tradename Nexavar®), Lapatinib (tradename Tykerb®) and/or therapeutic antibodies, e.g. Bevacizumab (tradename Avastin®) or cetuximab (tradename Erbitux®).

However, other treatments related to signalling pathways which fall under the scope of the present invention comprise the administration of BAY 43-9005, target receptors are VEGFR-2, VEGFR-3, c-KIT, PDGFR-B, RET and Raf-Kinase), BAY 57-9352 (target receptor is VEGFR-2), Sunitinib (tradename Sutent®, target receptors are VEGFR-1, VEGFR-2 and PDGFR), AG13925 (target receptors are VEGFR-1 and VEGFR-2), AG013736 (target receptors are VEGFR-1 and VEGFR-2), AZD2171 (target receptors are VEGFR-1 and VEGFR-2), ZD6474 (target receptors are VEGFR-1, VEGFR-2 and VEGFR-3), PTK-787/ZK-222584 (target receptors are VEGFR-1 and VEGFR-2), CEP-7055 (target receptors are VEGFR-1, VEGFR-2 and VEGFR-3), CP-547 (target receptors are VEGFR-1 and VEGFR-2), CP-632 (target receptors are VEGFR-1 and VEGFR-2), GW786024 (target receptors are VEGFR-1, VEGFR-2 and VEGFR-3), AMG706 (target receptors are VEGFR-1, VEGFR-2 and VEGFR-3), Imatinib mesylate (tradename Glivec®/Gleevec®, target receptors are bcr-abl and c-KIT), BMS-214662 (target enzyme is Ras farnesyl transferase), CCI-779 (target enzyme is mTOR), RAD0001 (tradename everolismus®, target enzyme is mTOR), CI-1040 (target enzyme is MEK),.

SU6668 (target receptors are VEGFR-2, PDGFR-B and FGFR-1),, AZD6126, CP547632 (target receptors are VEGFRs), CP868596 GW786034 (target receptors are PDGFRs), ABT-869 (target receptors are VEGFRs and PDGFRs), AEE788 (target receptors are VEGFRs and PDGFRs), AZD0530 (target enzymes are src and abl), and CEP7055.

In an preferred embodiment the genes encoding for estrogen receptor are selected from the group comprising ESR1 and/or ESR2. In an even more preferred embodiment the gene encoding for the estrogen receptor is ESR1.

Surprisingly, the inventors have found that the expression level of ESR1 has good prognostic and/or diagnostic value in gynecologic cancer, preferably ovarian cancer when tested before treatment, which resembles the de novo hormone activity of the tumor tissue. More surprisingly, the inventors have found that the benefit from chemotherapy was particularly striking in high grade and/or higher size tumors expressing estrogen and progesterone receptors, while the response of estrogen or progesterone receptor negative tumors remained to be poor.

Therefore, the inventors suggest for the first time, to use the expression level of ESR1 and /or ESR2 for the decision whether a given therapy is the most promising therapy for gynecologic cancer, preferably ovarian cancer, or if treatment modalities should be altered. As the inventors do show by comparing with the current standard techniques, these decisions cannot be drawn with e.g. IHC, as these techniques fail to determine the prognostic value of hormone receptors.

In another preferred embodiment the gene encoding for the progesterone receptor is PGR. In yet another preferred embodiment PGR is used for to decide on treatment modalities.

Moreover surprisingly, the inventors have found that the expression level of PGR has good prognostic and/or diagnostic value in gynecologic cancer, preferably ovarian cancer.

The inventors suggest for the first time, to use the expression level of PGR for the decision whether a given therapy is the most promising therapy for gynecologic cancer, preferably ovarian cancer or if treatment modalities should be altered.

In another preferred embodiment the gene encoding for the progesterone receptor is AR. In yet another preferred embodiment AR is used for to decide on treatment modalities.

Moreover surprisingly, the inventors have found that the expression level of AR has prognostic and/or diagnostic value in gynecologic cancer, preferably ovarian cancer.

The inventors suggest for the first time, to use the expression level of AR for the decision whether a given therapy is the most promising therapy for gynecologic cancer, preferably ovarian cancer or if treatment modalities should be altered.

Importantly, the decision when to use altered treatment modalities such as endocrine options can be influenced. These treatment modalities may be applied before, during or after chemotherapy and/or surgery.

In a preferred embodiment of the present invention, the methods of the present invention comprise comparing the level of mRNA expression of ESR1 and/or ESR2 and/or PGR and/or AR in a patient sample, and the average level of expression of ESR1 and/or ESR2 and/or PGR and/or AR in a sample from a control subject, e.g., a human subject without cancer. Comparison of the pattern of expression levels of ESR1 and/or ESR2 and/or PGR and/or AR can also be performed on any other reference.

In another preferred embodiment of the present invention, the methods of the present invention also comprise comparing the pattern of expression levels of mRNA of ESR1 and/or ESR2 and/or PGR and/or AR in an unclassified patient sample, and the pattern of expression levels of ESR1 and/or ESR2 and/or PGR and/or AR in a sample cohort comprising patients responding in different intensity to an administered neoadjuvant, adjuvant and /or palliative cancer therapy.

In a preferred embodiment of this invention the expression of ESR1 and/or ESR2 and/or PGR and/or AR can be utilized for discrimination of responders and non-responders to a given treatment, especially a chemotherapeutic intervention.

In a preferred embodiment of the present invention, it is provided that upregulated expression of said at least one gene encoding for a hormone receptor selected from the group comprising estrogen receptor, progesterone receptor and/or androgen receptor, especially of the RNA transcripts of ESR1, determined in step b) is indicative of a promising prediction as regards therapeutic success for a given mode of treatment.

Moreover, the combined analysis of estrogen, progesterone and androgen receptors improved the diagnostic value of the single marker evaluation, i.e. just based on estrogen, progesterone or androgen receptor.

By correlation analysis, the inventors have found that overexpression of ESR1 and preferably in untreated tumor samples, for example assessed by PCR analysis, indicates good prognosis of ovarian cancer patients treated by standard chemotherapy as indicated by prolonged disease free and overall survival. Especially a high expression of ESR1 was found to provide a good overall survival prognosis upon standard adjuvant chemotherapy. Also in the neoadjuvant chemotherapeutic setting the elevated expression level of estrogen recepors and progesterone receptors was associated with increased response. This indicates the direct link between treatment and directly related response, i.e. tumor shrinkage, whose assumption is difficult to draw in the adjuvant setting.

In other embodiments, intermediate expression of ESR1, for example assessed by PCR analysis, indicates poor prognosis of ovarian cancer patients treated by standard chemotherapy.

In a preferred embodiment of the present invention, it is provided that highly or intermediatly upregulated expression of said at least one gene encoding for a hormone receptor selected from the group comprising estrogen receptor, progesterone receptor and/or androgen receptor determined in step b) is indicative of a promising prediction as regards therapeutic success for a therapeutic regimen targeting hormone receptors selected from the group comprising estrogen receptor, progesterone receptor and/or androgen receptor especially endocrine treatment.

In another preferred embodiment of the present invention, it is provided that highly or intermediatly upregulated expression of said at least one gene encoding for the estrogen receptor especially ESR1 determined in step b) is indicative of a promising prediction as regards therapeutic success for a therapeutic regimen targeting the estrogen receptor, especially endocrine treatment.

For example, in the case of highly upregulated expression of said at least one gene encoding for the estrogen receptor especially ESR1 determined in step b) the nodal status may provide additional information with regard to outcome. In particular, if node negative the outcome of patients with high expression of e.g. ESR1 may be very good (i.e. above 95 % survival), whereas if node positive, the outcome may be inferior (i.e. at about 80% survival), while still being clearly superior to bad prognosis at low expression of e.g. ESR1 (i.e. at 22 % survival). This means that patients with tumors exhibiting high expression of e.g. ESR1 still may have a benefit from additional endocrine reatment.

In yet another preferred embodiment of the present invention, it is provided that low expression of said at least one gene encoding for a hormone receptor selected from the group comprising estrogen receptor, progesterone receptor and/or androgen receptor especially ESR1 indicates poor prognosis of gynecologic cancer, preferably ovarian cancer patients treated by standard chemotherapy.

Moreover this finding also enables to decide which patients should receive other treatment options targeting signalling pathways, e. g. small molecules.

In another preferred embodiment of the present invention, it is provided that the pattern of expression level(s) determined in step b) refers to a level of gene expression compared to a reference selected from the group comprising RPL37A, GAPDH, RPL13, and/or HPRT1. In a preferred embodiment these reference genes are RPL37A, GAPDH and HPRT1. In an even more preferred embodiment the reference genes are RPL37A and HPRT1. In an especially preferred embodiment of the present invention, it is provided that said reference or housekeeping gene is RPL37A.

Normalization to a housekeeping gene selected from the group comprising RPL37A, GAPDH, RPL13, and/or HPRT1 can provide the advantage of a highly reliable comparison.

In yet another preferred embodiment of the present invention, it is provided that said given mode of treatment acts on recruitment of lymphatic vessels, angiogenesis, cell proliferation, cell survival and/or cell motility, and/or comprises administration of a chemotherapeutic agent.

Furthermore, it is provided in an another preferred embodiment of the present invention that said given mode of treatment is selected from the group comprising chemotherapy, administration of small molecule inhibitors, antibody based regimen, anti-proliferation regimen, pro-apoptotic regimen, pro-differentiation regimen, radiation and/or surgical therapy. In yet other embodiments said given mode of treatment may include administration of cis-Platin (tradename Cis-platin®) .

Said chemotherapy may comprise the administration of at least one agent selected from the group comprising Cyclophosphamid (Endoxan®, Cyclostin®). Adriamycin (Doxorubicin) (Adriblastin®), BCNU (Carmustin) (Carmubris®), Busulfan (Myleran®), Bleomycin (Bleomycin®), Carboplatin (Carboplat®), Chlorambucil (Leukeran®), Cis-Platin (Cisplatin®), Platinex (Platiblastin®), Dacarbazin (DTIC®; Detimedac®), Docetaxel (Taxotere®), Epirubicin (Farmorubicin®), Etoposid (Vepesid®), 5-Fluorouracil (Fluroblastin®, Fluorouracil®), Gemcitabin (Gemzar®), Ifosfamid (Holoxan®), Interferon alpha (Roferon®), Irinotecan (CPT 11, Campto®), Melphalan (Alkeran®), Methotrexat (Methotrexat®, Farmitrexat®), Mitomycin C (Mitomycin®), Mitoxantron (Novantron®), Oxaliplatin (Eloxatine®), Paclitaxel (Taxol®), Prednimustin (Sterecyt®), Procarbazin (Natulan®), Pemetrexed (Alimta®), Ralitrexed (Tomudex®), Topotecan (Hycantin®), Trofosfamid (Ixoten®), Vinblastin (Velbe®), Vincristin (Vincristin®), Vindesin (Eldisine®) and/or Vinorelbin (Navelbine®).

In other embodiments said given mode of treatment may be endocrine treatment.

In a further aspect, the present invention provides a method of selecting a therapy modality for a patient afflicted with gynecologic cancer, preferably ovarian cancer, said method comprising the steps of:
a) obtaining a biological sample from said patient;
b) predicting from said sample, by the method according to any one of the aforementioned claims, therapeutic success for a plurality of individual modes of treatment; and
c) selecting a mode of treatment which is predicted to be successful in step b).

It is of note, that the inventors have proven the validity of the disclosed method in fresh tissue as well as fixed tissues. Also the inventors have shown the validity of the disclosed method in biopsies as well as tumor resectates.

On the basis of the findings of the present invention a therapy can be selected, which is most promising for the individual patient.

In addition the inventors suggest, for the first time, to use the expression level of a gene encoding for the estrogen receptor and/or progesterone receptor, especially ESR1, for the decision whether or not chemotherapeutic treatment should be kept as treatment or if endocrine treatment or treatment options targeting signalling pathways should be included as a treatment options.

In this regard, the accurate detection of the expression level of ESR1 enables to identify a subpopulation of tumors that overexpress ESR1 in an intermediate or slightly higher fashion, yet having a comparatively low overexpression of ESR1 that cannot be resolved by immunohistochemical techniques. This subpopulation may be particularly sensitive to endocrine treatment.

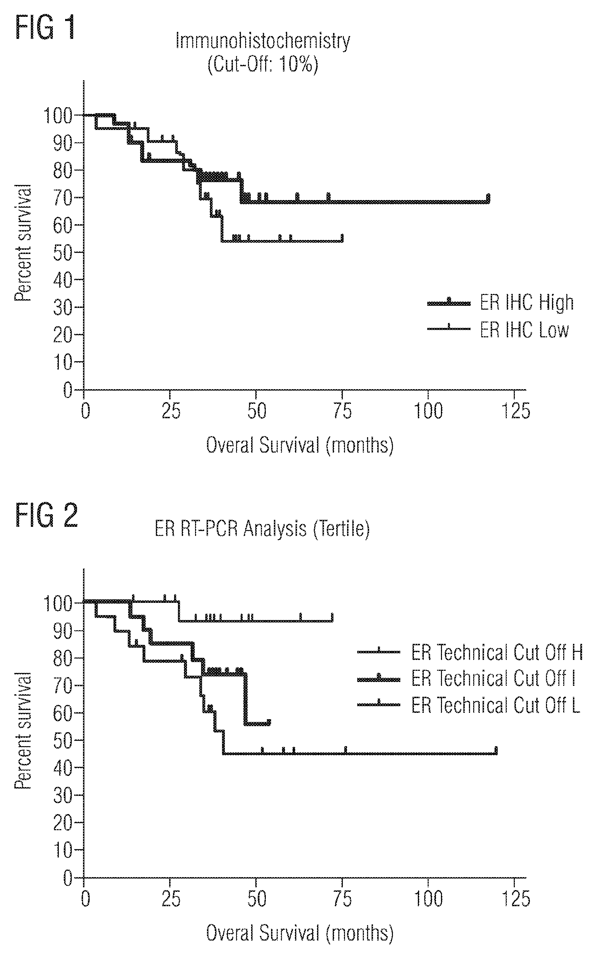

The method according to the invention may help to detect those gynecologic cancer patients, preferably ovarian cancer patients whose tumors are not susceptible to chemotherapeutic treatment or do need additional regimen and may be susceptible to endocrine treatment. These tumors have so far remained undetected with methods from the state of the art, as has also been shown by the inventors in the descrivbed patient cohort of this application (see Figure 1).

In a preferred embodiment, said method comprises in addition to step a) the following steps:
- obtaining a sample comprising cancer cells from said patient;
- separately maintaining aliquots of the sample in the presence of one or more test compositions;
- comparing expression of a single or plurality of molecules, selected from the ligands and/or receptors listed in Table 1 in each of the aliquots; and
- selecting a test composition which induces a lower level of expression of ligands and/or receptors from Table 1 and/or a higher level of expression of ligands and/or receptors from Table 1 in the aliquot containing that test composition, relative to the level of expression of each ligand in the aliquots containing the other test compositions.

The invention also provides a method for adapting therapeutic regimen based on individualized risk assessment for a patient suffering from or at risk of developing a gynecologic cancer, preferably ovarian cancer, comprising the steps of:
a) obtaining a biological sample from said patient;
b) determining, on a non protein basis, the expression level of at least one gene encoding for a hormone receptor selected from the group comprising estrogen receptor, progesterone receptor and/or androgen receptor in said sample;
c) comparing the pattern of expression level(s) determined in step b) with one or several reference pattern(s) of expression levels; and
d) implementing therapeutic regimen targeting hormone receptors selected from the group comprising estrogen receptor, progesterone receptor and/or androgen receptor or signalling pathways in said patient from the outcome of the comparison in step c).

The methods of the invention maybe used to evaluate a patient before, during and after therapy, for example to evaluate the reduction in tumor burden.

In the method of the present invention the determination of gene expression or the determination of the pattern of expression level is not limited to any specific method, or to the detection of mRNA.

It is particularly preferred that, in the method according to the invention, said expression level determined in step b) is determined by
a) a hybridization based method;
b) a PCR based method;
c) a method based on the electrochemical detection of particular molecules, and/or by
d) an array based method.

The above mentioned methods have in common that they are focussed on the detection of nucleic acids, particularly on the detection of mRNA, DNA, peptide nucleic acid (PNA), locked nucleic acid (LNA) and/or Morpholino.

Moreover, these methods provide the option that high quality determinations can be done as multiplex assays in one reaction based on the high specificity of the reagent design and performance.

Another advantage is that the method requires only small amounts of biological sample.

In yet another preferred embodiment of the present invention it is provided that said expression level of the RNA transcripts is determined by reverse transcriptase polymerase chain reaction (RT-PCR).

The method according to the invention has the advantage that it works on paraffin embedded tissues. In yet another preferred embodiment of the present invention it is provided that said expression level of the RNA transcripts is determined in formalin and/or paraffin fixed tissue samples.

For this purpose, at least one fixative may used in a preferred embodiment which is selected from the group consisting of Neutral Buffered Formaline, Unbuffered Formaline, Glutaraldehyde, Ethanol, Acetone, Methanol, Methacarn, Carnoy's fixative, AFA-Fixative (Formaldehyde, Ethanol and acetic acid), Pen-Fix (alcoholic formalin fixative), Glyo-Fixx (gly-oxal-based fixative), Hope (Hepes-glutamic acid buffer mediated organic solvent fixative), and/or Zinc Formal-Fixx (Formaldehyde fixative which contains zinc).

In yet another preferred embodiment of the present invention, it is provided that the expression level of at least one of the said ligands or receptors is determined in serum, plasma or whole blood samples.

In yet another preferred embodiment of the present invention, it is provided, that the information of the method disclosed herein is combines with standard histopathological data, such as TNM status, Grade, Location, Cell Type, Inflammatory status, to improve the validity of the result and/or adopt to the clinical situation.

In yet another preferred embodiment of the present invention, it is provided, that the results are adjusted to tumor cell content or sublocalization of the tissue material within the malignant tissue, e.g. invasive front, central oarts, angiogenic subregion, inflammatory region, etc.

Routinely, in tumor diagnosis tissue samples are taken as biopsies form a patient and undergo diagnostic procedures. For this purpose, the samples are fixed in formaline, embedded in paraffine and are then examined with immunohistochemistry methods. The formaline treatment leads to the inactivation of enzymes, as for example the ubiquitous RNA-digesting enzymes (RNAses). For this reason, the mRNA status of the tissue (the so called transcriptome), remains unaffected.

However, the formaline treatment leads to partial depolymerization of the individual mRNA molecules. Same applies for other fixatives, as for example mentioned in the above enumeration.

For this reason, it is provided in a preferred embodiment of the present invention that after lysis, the sample is treated with silica-coated magnetic particles and a chaotropic salt, for purification of the nucleic acids contained in said sample for further determination.

However the isolation method may alternatively also be silica column based with or without chaotropic agents.

Collaborators of the inventors of the present invention have developed an approach which however allows successful purification of mRNA out of tissue samples fixed in such manner, and which is disclosed, among others, in WO03058649, WO2006136314A1 and DE10201084A1, the content of which is incorporated herein by reference.

Said method comprises the use of magnetic particles coated with silica (SiO₂). The silica layer is closed and tight and is characterized by having an extremely small thickness on the scale of a few nanometers. These particles are produced by an improved method that leads to a product having a closed silica layer and thus entail a highly improved purity. The said method prevents an uncontrolled formation of aggregates and clusters of silicates on the magnetite surface whereby positively influencing the additional cited properties and biological applications. The said magnetic particles exhibit an optimized magnetization and suspension behavior as well as a very advantageous run-off behavior from plastic surfaces. These highly pure magnetic particles coated with silicon dioxide are used for isolating nucleic acids, including DNA and RNA, from cell and tissue samples, the separating out from a sample matrix ensuing by means of magnetic fields. These particles are particularly well-suited for the automatic purification of nucleic acids, mostly from biological body samples for the purpose of detecting them with different amplification methods.

The selective binding of these nucleic acids to the surface of said particles is due to the affinity of negatively charged nucleic acids to silica containing media in the presence of chaotropic salts like guanidinisothiocyanate. Said binding properties are known as the so called "boom principle". They are described in the European patent EP819696, the content of which is incorporated herein by reference.

The said approach is particularly useful for the purification of mRNA out of formaline and/or paraffine fixed tissue samples. In contrast to most other approaches, which leave very small fragments behind that are not suitable for later determination by PCR and/or hybridization technologies, the said approach creates mRNA fragments which are large enough to allow specific primer hybridzation and/or specific probe hybridization. A minimal size of at least 50 bp, more preferably 100 bp, even more preferably 200 base pairs is needed for specific and robust detection of target gene expression. Moreover it is also necessary to not have too many inter-sample variations with regard to the size of the RNA fragments to guarantee comparability of gene expression results. Other issues of perturbance of expression data by sample preparation problems relate to the contamination level with DNA, which is lower compared to other bead or column based technologies.

The said approach thus allows a highly specific determination of the status of hormone receptors selected from the group comprising estrogen receptor, progesterone receptor and/or androgen receptor with one of the above introduced methods, particularly with hybridization based methods, PCR based methods and/or array based methods, even in fixed routine tissue samples, and is thus extremely beneficial in the context of the present invention, as it allows the use of tissue samples fixed with formaline and/or paraffine, which are available in tissue banks and connected to clinical databases of sufficient follow-up to allow retrospective analysis.

Another important aspect is that the said approach allows the simultaneous determination of more than one analyte (multiplexing), and is thus ideally suited for the determination of hormone receptors selected from the group comprising estrogen receptor, progesterone receptor and/or androgen receptor especially ESR1, ESR2, PGR and/or of one or more housekeeping genes in said sample. Alternatively to housekeeping genes, which are per definition being expressed in virtually all cells to similar amounts, tumor specific, endothelial cell specific and or stroma specific genes may be included to further increase the diagnostic precision of said method. By this approach one can derive a calibration factor in order to normalize the expression values of the target genes in samples which have different shares of tumor tissue and non tumor tissue.

In a preferred embodiment of the present invention, it is provided that said gynecologic cancer is selected from the group comprising breast cancer, ovarian cancer, vulvar cancer, vaginal cancer, tubal cancer, endometrian cancer and/or cervical cancer.

In a more preferred embodiment of the present invention, it is provided that said gynecologic cancer is ovarian cancer.

In yet another preferred embodiment of the present invention, it is provided that said endocrine treatment is a hormonal treatment and/or antihormonal treatment.

Said endocrine treatment may comprises the administration of antagonists of estrogen binding to the estrogen receptor, estrogen reuptake inhibitors, selective estrogen receptor downregulators, or as inhibitors of estrogen biosynthesis, such as aromatase inhibitors. Said endocrine treatment may also comprise similiar approaches to target progesterone and/or androgen receptors.

Said endocrine treatment may include administration of hormones or hormone analogs, synthetic hormones or other drugs to the patient, e.g. tamoxifen and/or gosereline (tradename Zoladex®). In a preferred embodiment the said endocrine treatment comprises the administration of tamoxifen or of tamoxifen and gosereline.

In other preferred embodiments said endocrine treatment may comprise the administration of an anti estrogen drug selected from the group comprising anastrozole, letrozole, exemestane, fulvestrant, toremifene and megasterol acetate.

In yet another preferred embodiments said endocrine treatment may comprise the administration of estrogen, progestin and/or gestagen.

In yet another embodiment of the invention a method for correlating the clinical outcome of a patient suffering from or at risk of developing a gynecologic cancer, preferably ovarian cancer with the presence or non-presence of a defect in expression levels of the RNA transcripts of at least one gene encoding for a hormone receptor selected from the group comprising estrogen receptor, progesterone receptor and/or androgen receptor is provided, said method comprising the steps of:
a) obtaining a fixed biological sample from said patient;
b) determining the expression levels of the RNA transcripts of at least one gene encoding for a hormone receptor selected from the group comprising estrogen receptor, progesterone receptor and/or androgen receptor, and
c) correlating the pattern of expression level(s) determined in b) with said patient's data, said data being selected from the group consisting of etiopathology data, clinical symptoms, anamnesis data and/or data concerning the therapeutic regimen.

The said method is particularly beneficial for epidemiological studies. These studies profit from the fact that large tissue databases exist comprising paraffin and/or formalin fixed tissue samples together with an extensive documentation of the patient's history, including etiopathology data, clinical symptoms, anamnesis data and/or data concerning the therapeutic regimen. The said methods advantageously allows for large scale studies.

In another preferred embodiment of the present invention, a kit useful for carrying out a method of any one of the aforementioned claims, comprising at least a pair of gene specific primers and/or probes each having a sequence sufficiently complementary to at least one gene or gene fragments or genomic nucleic acid sequence encoding for a at least one gene coding for a hormone receptor selected from the group comprising estrogen receptor, progesterone receptor and/or androgen receptor for quantifying the expression of said at least one gene or gene fragment or genomic nucleic acid sequence, and/or their fractions, variants, homologues, derivatives, fragments, complements, hybridizing counterparts, or molecules sharing a sequence identity of at least 70%, preferably 95 %.

These nucleic acids can be used either as primers for a polymerase chain reaction protocol, or as detectable probes for monitoring the said process.

Furthermore it is provided that the said nucleic acid or nucleic acid homolouge is selected from the group consisting of DNA, RNA, PNA, LNA and/or Morpholino. The nucleic acid may, in a preferred embodiment, be labelled with at least one detectable marker. This feature is applicable particularly for those nucleic acids which serve as detectable probes for monitoring the polymerase chain reaction process.

Such detectable markers may for example comprise at least one label selected from the group consisting of fluorescent molecules, luminescent molecules, radioactive molecules, enzymatic molecules and/or quenching molecules.

In a particularly preferred embodiment, the said detectable probes are labeled with a fluorescent marker at one end and a quencher of fluorescence at the opposite end of the probe. The close proximity of the reporter to the quencher prevents detection of its fluorescence; breakdown of the probe by the 5' to 3' exonuclease activity of the taq polymerase breaks the reporter-quencher proximity and thus allows unquenched emission of fluorescence, which can be detected. An increase in the product targeted by the reporter probe at each PCR cycle therefore causes a proportional increase in fluorescence due to the breakdown of the probe and release of the reporter.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

**Table 1: Genes of interest**

| Gene_Symbol [A] | Ref. Sequences De scription [A] | Ref. Sequences [A] | Unigene_ID [A] |
|---|---|---|---|
| ESR1 | Estrogen receptor | NM 000125.2 | Hs.208124 |
| | | | Hs.525392; |
| ESR2 | Estrogen receptor | NM 001040276+1 | HS660607 |
| | | | Hs.525392; |
| ESR2 | Estrogen receptor | NM 001040276+1 | HS660607 |
| | | | Hs.525392; |
| ESR2 | Estrogen receptor | NM 001437+2 | HS660607 |
| PGR | Progesterone receptor | NM 000926.4 | Hs.368072 |
| AR | Androgen receptor | NM 000044+2 | Hs.496240 |
| AR | Androgen receptor | NM 001011645+1 | Hs.496240 |

The terms "Ref. Sequences" and "Unigene_ID" relate to databases in which the respective proteins are listed under the given access number. These databases can be accessed over the NCBI server.

### Brief description of the examples and drawings

Additional details, features, characteristics and advantages of the object of the invention are disclosed in the subclaims, and the following description of the respective figures and examples, which, in an exemplary fashion, show preferred embodiments of the present invention. However, these examples should by no means be understood as to limit the scope of the invention.
- Figure 1: Kaplan-Meier-Analysis of overall survival (OAS) of patients suffering from ovarian cancer based on im- munohistochemistry for ESR1 (ER) determination. A cut off at 10% % cells being positive for ER stain- ing is depicted. The overall survival (OAS) is de- picted in months.
- Figure 2: Kaplan-Meier-Analysis of overall survival (OAS) of patients suffering from ovarian cancer based on relative gene expression of ESR1 as determined by RT-PCR. The patient cohort was divided by technical cut off setting, meaning the unbiased tertiles were used to built three equally sized groups of pa- tients. ESR1 high expressing tumors are depicted as "ER Technical CutOff H" with "H" meaning "High". ESR1 intermediate expressing tumors are depicted as "ER Technical CutOff I" with "I" meaning "Interme- diate". ESR1 low expressing tumors are depicted as "ER Technical CutOff L" with "L" meaning "Low". The overall survival (OAS) is depicted in months.

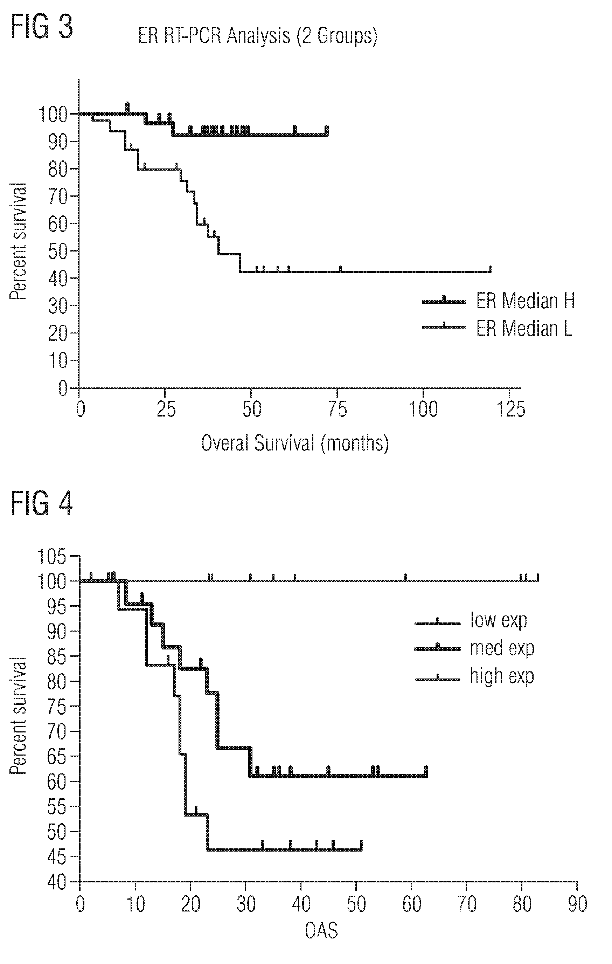

- Figure 3: Kaplan-Meier-Analysis of overall survival (OAS) of patients suffering from ovarian cancer based on relative gene expression of ESR1 as determined by RT-PCR. The patient cohort was divided by technical cut off setting, meaning the unbiased median was used to built two equally sized groups of patients. ESR1 high expressing tumors are depicted as "ER Me- dian H" with "H" meaning "High". ESR1 intermediate expressing tumors are depicted as "ER Median L" with "L" meaning "Low". The overall survival (OAS) is depicted in months.
- Figure 4: Kaplan-Meier-Analysis of overall survival (OAS) of patients suffering from ovarian cancer based on relative gene expression of PGR as determined by RT-PCR. The patient cohort was divided by technical cut off setting, meaning the unbiased tertiles were used to built three equally sized groups of pa- tients. PGR high expressing tumors are depicted as "high exp". PGR intermediate expressing tumors are depicted as "med exp". PGR low expressing tumors are depicted as "low exp". The overall survival (OAS) is depicted in months.

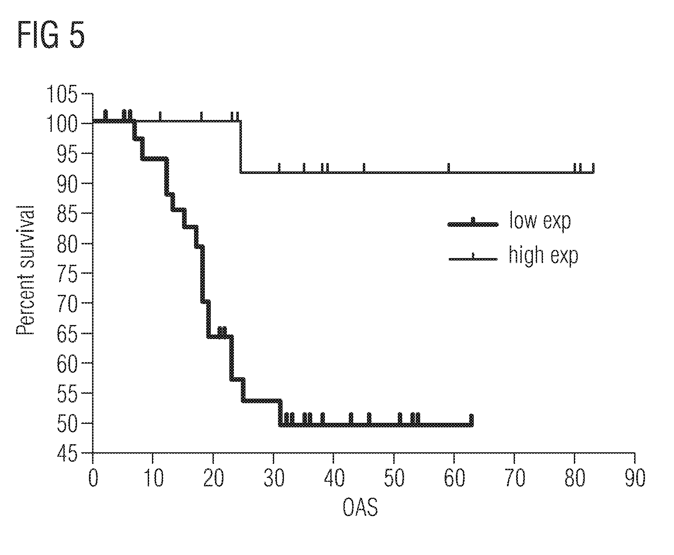

- Figure 5: Kaplan-Meier-Analysis of overall survival (OAS) of patients suffering from ovarian cancer based on relative gene expression of PGR as determined by RT-PCR. The patient cohort was divided by technical cut off setting, meaning the unbiased median was used to built two equally sized groups of patients. PGR high expressing tumors are depicted as "high exp". PGR low expressing tumors are depicted as "low exp". The overall survival (OAS) is depicted in months.

### Patients for ESR1 determination:

Patients (n=57) selected within a non-stratified, population based ovarian cancer patient cohort for this study had undergone ovarian surgery. 84.2 % of patients were under platinum based chemotherapy, 1.8 % received other chemotherapy while 1.8 % received no chemotherapy. The following table 2 shows the patient data:

**Table 2: patient data for ESR1 determination:**

| | | | |
|---|---|---|---|
| Cases | | 57 | |
| Median age (years) | | 55 | |
| | Range | 36 - 81 | |
| Median follow up (months) | | 37 | |
| | Range | 4 - 118 | |

| Death during follow up | | | |
|---|---|---|---|
| | still alive | 41 | 71.9% |
| | Dead | 16 | 28.1% |
| | | | |

| Feature | | Cases | Percent |
|---|---|---|---|
| | | | |

| Age at surgery (years) | | | |
|---|---|---|---|
| | ≤ 60 | 37 | 64.9% |
| | > 60 | 20 | 35,1% |

| Hislogical type | | | |
|---|---|---|---|
| | Serous | 39 | 68.4% |
| | Mucinous | 2 | 3.5% |
| | endometroid | 6 | 10.5% |
| | clear cell | 3 | 5.3% |
| | transitional cell | 2 | 3.5% |
| | undifferentiated | 5 | 8.8% |

| FIGO stage | | | |
|---|---|---|---|
| | I | 9 | 15.8% |
| | II | 6 | 10.5% |
| | III | 39 | 68.4% |
| | IV | 3 | 5.3% |

| pT | | | |
|---|---|---|---|
| | pT1 | 10 | 17.5% |
| | pT2 | 6 | 10.5% |
| | pT3 | 41 | 71.9% |

| pN | | | |
|---|---|---|---|
| | pN0 | 29 | 50.9% |
| | pN1 | 15 | 26.3% |
| | pNX | 13 | 22.8% |
| Silverberg | grade | | |
| | G1 | 5 | 8.8% |
| | G2 | 27 | 47.4% |
| | G3 | 25 | 43.9% |

| Residual tumor after surgery | | | |
|---|---|---|---|
| | None | 21 | 36.8% |
| | < 2 cm | 12 | 21.1% |
| | > 2 cm | 6 | 10.5% |
| | Missing | 18 | 31.6% |

| Chemotherapy (CTX) | | | |
|---|---|---|---|
| | Platinum based | 48 | 84.2% |
| | other CTX | 1 | 1.8% |
| | no CTX | 1 | 1.8% |
| | Missing | 7 | 12.3% |

### Patients for PGR determination:

Patients (n=60) selected within a non-stratified, population based ovarian cancer patient cohort for this study had undergone ovarian surgery. 86.7 % of patients were under platinum based chemotherapy, 1.7 % received other chemotherapy while 5.0 % received no chemotherapy. The following table 3 shows the patient data:

**Table 3: patient data for PGR determination:**

| | | | |
|---|---|---|---|
| Cases | | 60 | |

| Median age (years) | | 59 | |
|---|---|---|---|
| | Range | 34 - 80 | |

| Median follow up (months) | | 30 | |
|---|---|---|---|
| | Range | 2 - 81 | |

| Death during follow up | | | |
|---|---|---|---|
| | still alive | 44 | 73.3% |
| | dead | 16 | 26.7% |
| | | | |

| Feature | | Cases | Percent |
|---|---|---|---|
| Age at surgery (years) | | | |
| | = 60 | 32 | 53.3% |
| | > 60 | 28 | 46.7% |

| Hislogical type | | | |
|---|---|---|---|
| | serous | 41 | 68.3% |
| | mucinous | 2 | 3.3% |
| | endometroid | 8 | 13.3% |
| | clear cell | 0 | 0.0% |
| | transitional cell | 3 | 5.0% |
| | undifferentiated | 6 | 10.0% |

| FIGO stage | | | |
|---|---|---|---|
| | I | 12 | 20.0% |
| | II | 3 | 5.0% |
| | III | 41 | 68,3% |
| | IV | 4 | 6.7% |

| pT | | | |
|---|---|---|---|
| | pT1 | 13 | 21.7% |
| | pT2 | 5 | 8.3% |
| | pT3 | 42 | 70.0% |

| pN | | | |
|---|---|---|---|
| | pN0 | 16 | 26.7% |
| | pN1 | 30 | 50.0% |
| | pNX | 14 | 23.3% |

| Silverberg grade | | | |
|---|---|---|---|
| | G1 | 10 | 16.7% |
| | G2 | 23 | 38.3% |
| | G3 | 27 | 45.0% |

| Residual tumor after surgery | | | |
|---|---|---|---|
| | none | 31 | 51.7% |
| | < 2 cm | 7 | 11.7% |
| | > 2 cm | 5 | 8.3% |
| | missing | 17 | 28.3% |

| Chemotherapy (CTX) | | | |
|---|---|---|---|
| | Platinum based | 52 | 86.7% |
| | other CTX | 1 | 1.7% |
| | no CTX | 3 | 5.0% |
| | missing | 4 | 6.7% |

### Example 1

### IHC approach

For the IHC approach, ESR1 levels were assessed on a protein basis in 57 patients of the non-stratified, population based ovarian cancer patient cohort as shown in table 2, using an antibody on the commercially available DAKO kit on a Ventana autostainer.

Immunoreactivity was evaluated in the tumor cells based on the staining intensity and percentage of cells being positive by two independent pathologists.

The IHC analysis of the ESR1 levels did not reveal statistical significance (Hazard ratio (HR)=0.67; p=0.42). Fig. 1 shows the Kaplan-Meier-Analysis based on the immunohistochemistry for ESR1 (ER) determination. A cut off at 10% % cells being positive for ER staining is depicted.

In the examples ER and ESR1 are used synonymous.

Interestingly as can be seen in Figure 1, by taking the 10% positively stained cells as a cut-off for immunohistochemistry, resulting in two almost equally sized patient groups, no difference could be seen with regard to the survival (HR 0.67; 95% CI=0.24-1.81; p=0.42). High expression of ESR1 was observed in 32 patients, low expression of ESR1 was observed in 23 patients.

Kinetic PCR (kPCR) determination of ESR1 and PGR RNA expression displayed a broad range of relative copy number (2.5 logs).

### Example 2

### Determination of ESR1 expression using RT-PCR

ESR1 levels were assessed in 57 patients of the non-stratified, population based ovarian cancer patient cohort as shown in table 2.

### Molecular methods

RNA was isolated from formalin-fixed paraffin-embedded ("FFPE") tumor tissue samples employing an experimental method based on proprietary magnetic beads from Siemens Medical Solutions Diagnostics. In short, the FFPE slide were lysed and treated with Proteinase K for 2 hours 55°C with shaking. After adding a binding buffer and the magnetic particles (Siemens Medical Solutions Diagnostic GmbH, Leverkusen, Germany) nucleic acids were bound to the particles within 15 minutes at room temperature. On a magnetic stand the supernatant was taken away and beads were washed several times with washing buffer. After adding elution buffer and incubating for 10 min at 70°C the supernatant was taken away on a magnetic stand without touching the beads. After normal DNAse I treatment for 30 min at 37°C and inactivation of DNAse I the solution was used for reverse transcription-polymerase chain reaction (RT-PCR).

RT-PCR was run as standard kinetic one-step Reverse Transcriptase TaqMan™ polymerase chain reaction (RT-PCR) analysis on a ABI7900 (Applied Biosystems) PCR system for assessment of mRNA expression. Raw data of the RT-PCR were normalized to one or combinations of the housekeeping genes RPL37A, GAPDH, RPL13, and HPRT1 by using the comparative ΔΔCT method, known to those skilled in the art. In brief, a total of 40 cycles of RNA amplification were applied and the cycle threshold (CT) of the target genes was set as being 0.5. CT scores were normalized by subtracting the CT score of the housekeeping gene RPL37A or the mean of the combinations from the CT score of the target gene (Delta CT). RNA results were then reported as 40-Delta CT or 2^{((40-(CT Target Gene - CT Housekeeping Gene)*(-1)))} (2^ (40-(CT Target Gene - CT Housekeeping Gene)*(-1))) scores, which would correlate proportionally to the mRNA expression level of the target gene. For each gene specific Primer/Probe were designed by Primer Express ® software v2.0 (Applied Biosystems) according to manufacturers instructions.

### Statistics

The statistical analysis was performed with Graph Pad Prism Version 4 (Graph Pad Prism Software, Inc).

The clinical and biological variables were categorised into normal and pathological values according to standard norms. The Chi-square test was used to compare different groups for categorical variables. To examine correlations between different molecular factors, the Spearman rank correlation coefficient test was used.

For univariate analysis, logistic regression models with one covariate were used when looking at categorical outcomes. Survival curves were estimated by the method of Kaplan and Meier, and the curves were compared according to one factor by the log rank test. For the estimation of multivariate models, all parameters which were significant at the univariate analysis (*p*<0.05) were fitted to a Cox regression model using a backward forward stepwise method for the selection of co-variates. Confidence intervals (CI) at 95% for hazard rates (HR) were calculated. All the probabilities that were calculated were two-tailed.

Interestingly as can be seen in Figure 2, by taking the tertile as technical cut-off, three highly significantly different groups in terms of outcome could be constructed, wherein expression of ESR1 was defined as high, median and low according to values above and up to the median, respectively: a favourable group "ER Technical Cut OFF H", consisting of patients with high expression of ESR1 ( undefined median survival time); an intermediate group "ER Technical Cut OFF I", consisting of patients with median expression of ESR1 (undefined median survival time) and finally a poor prognosis group "ER Technical Cut OFF L", consisting of patients with low expression of ESR1 (median survival time 40 months; Hazard Ratio 6.84; p=0.0089). High expression of ESR1 was observed in 18 patients with 1 cancer related death occurring and was found to be a significant positive prognostic factor for overall survival. Intermediate expression of ESR1 was observed in 20 patients with 6 cancer related deaths occurring. Low expression of ESR1 was observed in 19 patients with 8 cancer related deaths occurring and was found to be a significant negative prognostic factor for overall survival. The Kaplan Meier curve is presented in Fig. 2.

Interestingly as can be seen in Figure 3, by taking the median as technical cut-off, two highly significantly different groups in terms of outcome could be constructed, wherein expression of ESR1 was defined as high and low according to values above and up to the median, respectively: a favourable group "ER Median H", consisting of patients with high expression of ESR1 ( undefined median survival time); an a poor prognosis group "ER Median L", consisting of patients with low expression of ESR1 (median survival time 40 months; Hazard Ratio 7.83; 95% CI=1.91-13.73; p=0.0012). High expression of ESR1 was observed in 29 patients with 2 cancer related deaths occurring and was found to be a significant positive prognostic factor for overall survival. Low expression of ESR1 was observed in 28 patients with 14 cancer related deaths occurring and was found to be a significant negative prognostic factor for overall survival. The Kaplan Meier curve is presented in Fig. 3.

As exemplified in Figure 2 and in Figure 3, it could be shown that high expression of ESR1 was related to prolonged survival as exhibited by a Hazard ratio 7.8; Chi square 10.5 , p=0.0012 and Chi square 6.84 , p=0.0089 respectively. Treatment options for those patients with poor expectation to survive could be to keep standard treatment.

Interestingly, the intermediate expression tertile did show intermediate survival. This group may benefit most from endocrine treatment options.

Treatment options for those patients with poor expectation to survive could draw substantial benefit from alternative treatment options like targeting signalling pathways, e.g. small molecules like Sunitinib (tradename Sutent®), Sorafenib (tradename Nexavar®), Lapatinib (Tykerb®) and/or therapeutic antibodies, e.g. Bevacizumab (tradename Avastin®) or cetuximab (tradename Erbitux®).

### Example 3

### Determination of PGR expression using RT-PCR

PGR levels were assessed in 60 patients of the non-stratified, population based ovarian cancer patient cohort as shown in table 3.

RNA was isolated from formalin-fixed paraffin-embedded ("FFPE") tumor tissue samples employing an experimental method based on proprietary magnetic beads from Siemens Medical Solutions Diagnostics. In short, the FFPE slide were lysed and treated with Proteinase K for 2 hours 55°C with shaking. After adding a binding buffer and the magnetic particles (Siemens Medical Solutions Diagnostic GmbH, Leverkusen, Germany) nucleic acids were bound to the particles within 15 minutes at room temperature. On a magnetic stand the supernatant was taken away and beads were washed several times with washing buffer. After adding elution buffer and incubating for 10 min at 70°C the supernatant was taken away on a magnetic stand without touching the beads. After normal DNAse I treatment for 30 min at 37°C and inactivation of DNAse I the solution was used for reverse transcription-polymerase chain reaction (RT-PCR).

RT-PCR was run as standard kinetic one-step Reverse Transcriptase TaqMan™ polymerase chain reaction (RT-PCR) analysis on a ABI7900 (Applied Biosystems) PCR system for assessment of mRNA expression. Raw data of the RT-PCR were normalized to one or combinations of the housekeeping genes RPL37A, GAPDH, RPL13, and HPRT1 by using the comparative ΔΔCT method, known to those skilled in the art. In brief, a total of 40 cycles of RNA amplification were applied and the cycle threshold (CT) of the target genes was set as being 0.5. CT scores were normalized by subtracting the CT score of the housekeeping gene RPL37A or the mean of the combinations from the CT score of the target gene (Delta CT). RNA results were then reported as 40-Delta CT or 2^{((40-(CT Target Gene - CT Housekeeping Gene)*(-1)))} scores, which would correlate proportionally to the mRNA expression level of the target gene. For each gene specific Primer/Probe were designed by Primer Express ® software v2.0 (Applied Biosystems) according to manufacturers instructions.

### Statistics

The statistical analysis was performed with Graph Pad Prism Version 4 (Graph Pad Prism Software, Inc).

The clinical and biological variables were categorised into normal and pathological values according to standard norms. The Chi-square test was used to compare different groups for categorical variables. To examine correlations between different molecular factors, the Spearman rank correlation coefficient test was used.

For univariate analysis, logistic regression models with one covariate were used when looking at categorical outcomes. Survival curves were estimated by the method of Kaplan and Meier, and the curves were compared according to one factor by the log rank test. For the estimation of multivariate models, all parameters which were significant at the univariate analysis (*p*<0.05) were fitted to a Cox regression model using a backward forward stepwise method for the selection of co-variates. Confidence intervals (CI) at 95% for hazard rates (HR) were calculated. All the probabilities that were calculated were two-tailed.

Interestingly as can be seen in Figure 4, by taking the tertile as technical cut-off, three highly significantly different groups in terms of outcome could be constructed, wherein expression of PGR was defined as high, median and low according to values above and up to the median, respectively: a favourable group "high exp", consisting of patients with high expression of PGR; an intermediate group "med exp", consisting of patients with median expression of PGR and finally a poor prognosis group "low exp", consisting of patients with low expression of PGR (Hazard Ratio=7.34; p=0.0068). The Kaplan Meier curve is presented in Fig. 4.

Interestingly as can be seen in Figure 5, by taking the median as technical cut-off, two highly significantly different groups in terms of outcome could be constructed, wherein expression of PGR was defined as high and low according to values above and up to the median, respectively: a favourable group "high exp", consisting of patients with high expression of PGR and a poor prognosis group "low exp", consisting of patients with low expression of PGR (Hazard Ratio 10.56; 95% CI=1.6-14.49; p=0.0038). The Kaplan Meier curve is presented in Fig. 5.

### Prognostic factors for survival

High expression of ESR1 and PGR as determined by PCR technologies was associated with a significantly improved survival (see Fig. 2 -5).

It is obvious that patients with a tumor being determined as having high expression of ESR1 or PGR have a much better expectation to survive than those having a tumor being determined as having low expression of ESR1 or PGR.

The shown differentiation was not possible with IHC methods, only with the approach according to the present invention, as shown by the comparative IHC approach as shown as example 1.

The immunohistochemistry failed to reveal any prognostic value of ESR1 immunreactivity within the very same cohort and tissue sample material.

## Claims

1. A method for predicting a clinical response of a patient suffering from or at risk of developing a gynecologic cancer towards a given mode of treatment, said method comprising the steps of:
a) obtaining a biological sample from said patient;
b) determining, on a non protein basis, the expression level of at least one gene encoding for a hormone receptor selected from the group comprising estrogen receptor, progesterone receptor and/or androgen receptor in said sample;
c) comparing the pattern of expression level(s) determined in step b) with one or several reference pattern(s) of expression levels; and
d) predicting therapeutic success for said given mode of treatment in said patient from the outcome of the comparison in step c).

2. A method for predicting a clinical response of a patient suffering from or at risk of developing a gynecologic cancer towards a given mode of treatment, said method comprising the steps of:
a) obtaining a biological sample from said patient;
b) determining, on a non protein basis, the expression level of at least one gene encoding for a hormone receptor selected from the group comprising estrogen receptor, progesterone receptor and/or androgen receptor in said sample;
c) comparing the pattern of expression level(s) determined in step b) with one or several reference pattern(s) of expression levels; and
d) predicting therapeutic success for a therapeutic regimen targeting hormone receptors selected from the group comprising estrogen receptor, progesterone receptor and/or androgen receptor or signalling pathways in said patient from the outcome of the comparison in step c).

3. The method according to claim 1 or 2, **characterized in that** the gene encoding for the estrogen receptor is ESR1.

4. The method according to any one of the aforementioned claims, wherein upregulated expression of said at least one gene encoding for a hormone receptor selected from the group comprising estrogen receptor, progesterone receptor and/or androgen receptor determined in step b) is indicative of a promising prediction as regards therapeutic success for said given mode of treatment.

5. The method according to any one of the aforementioned claims, wherein intermediate upregulated expression of said at least one gene encoding for a hormone receptor selected from the group comprising estrogen receptor, progesterone receptor and/or androgen receptor determined in step b) is indicative of a promising prediction as regards therapeutic success for a therapeutic regimen targeting hormone receptors selected from the group comprising estrogen receptor, progesterone receptor and/or androgen receptor.

6. The method according to any one of the aforementioned claims, wherein the pattern of expression level(s) determined in step b) refers to a level of gene expression compared to a reference selected from the group comprising RPL37A, GAPDH, RPL13, and/or HPRT1.

7. The method according to any one of the aforementioned claims, wherein said given mode of treatment acts on recruitment of lymphatic vessels, angiogenesis, cell proliferation, cell survival and/or cell motility, and/or comprises administration of a chemotherapeutic agent.

8. The method according to any one of the aforementioned claims, **characterized in that** said given mode of treatment is selected from the group comprising chemotherapy, administration of small molecule inhibitors, antibody based regimen, anti-proliferation regimen, pro-apoptotic regimen, pro-differentiation regimen, radiation and/or surgical therapy.

9. A method of selecting a therapy modality for a patient afflicted with a gynecologic cancer, said method comprising the steps of:
a) obtaining a biological sample from said patient;
b) predicting from said sample, by the method according to any one of the aforementioned claims, therapeutic success for a plurality of individual modes of treatment; and
c) selecting a mode of treatment which is predicted to be successful in step b).

10. A method for adapting therapeutic regimen based on individualized risk assessment for a patient suffering from or at risk of developing a gynecologic cancer, comprising the steps of:
a) obtaining a biological sample from said patient;
b) determining, on a non protein basis, the expression level of at least one gene encoding for a hormone receptor selected from the group comprising estrogen receptor, progesterone receptor and/or androgen receptor in said sample;
c) comparing the pattern of expression level(s) determined in step b) with one or several reference pattern(s) of expression levels; and
d) implementing therapeutic regimen targeting hormone receptors selected from the group comprising estrogen receptor, progesterone receptor and/or androgen receptor or signalling pathways in said patient from the outcome of the comparison in step c).

11. The method according to any one of the aforementioned claims, wherein said expression level determined in step b) is determined by
a) a hybridization based method;
b) a PCR based method;
c) a method based on the electrochemical detection of particular molecules, and/or by
d) an array based method.

12. The method according to any one of the aforementioned claims, **characterized in that** said expression level of the RNA transcripts is determined by reverse transcriptase polymerase chain reaction.

13. The method according to any one of the aforementioned claims, **characterized in that** said expression level of the RNA transcripts is determined in formalin and/or paraffin fixed tissue samples.

14. The method according to any one of the aforementioned claims, wherein, after lysis, the sample is treated with silica-coated magnetic particles and a chaotropic salt, for purification of the nucleic acids contained in said sample for further determination.

15. The method according to any one of the aforementioned claims **characterized in that** said gynecologic cancer is selected from the group comprising breast cancer, ovarian cancer, vulvar cancer, vaginal cancer, tubal cancer, endometrian cancer and/or cervical cancer.

16. The method according to any one of the aforementioned claims **characterized in that** said gynecologic cancer is ovarian cancer.

17. The method according to any one of the aforementioned claims, **characterized in that** said endocrine treatment is a hormonal treatment and/or antihormonal treatment.

18. The method according to any one of the aforementioned claims, **characterized in that** said endocrine treatment comprises the administration of tamoxifen.

19. A method for correlating the clinical outcome of a patient suffering from or at risk of developing a gynecologic cancer with the presence or non-presence of a defect in expression levels of the RNA transcripts of at least one gene encoding for a hormone receptor selected from the group comprising estrogen receptor, progesterone receptor and/or androgen receptor, said method comprising the steps of:
a) obtaining a fixed biological sample from said patient;
b) determining the expression levels of the RNA transcripts of at least one gene encoding for a hormone receptor selected from the group comprising estrogen receptor, progesterone receptor and/or androgen receptor, and
c) correlating the pattern of expression level(s) determined in b) with said patient's data, said data being selected from the group consisting of etiopathology data, clinical symptoms, anamnesis data and/or data concerning the therapeutic regimen.

20. A kit useful for carrying out a method of any one of the aforementioned claims, comprising at least a pair of gene specific primers and/or probes each having a sequence sufficiently complementary to at least one gene or gene fragments or genomic nucleic acid sequence encoding for a at least one gene coding for a hormone receptor selected from the group comprising estrogen receptor, progesterone receptor and/or androgen receptor for quantifying the expression of said at least one gene or gene fragment or genomic nucleic acid sequence, and/or their fractions, variants, homologues, derivatives, fragments, complements, hybridizing counterparts, or molecules sharing a sequence identity of at least 70%, preferably 95 %.
